# EUROPEAN PATENT APPLICATION

(11) **EP 2 002 785 A2**
(43) Date of publication of application: **17.12.2008**
(21) Application number: 07738374.3
(22) Date of filing: 13.03.2007
(51) Int. Cl.: A61B 5/022, F16L 37/00

(54) **BLOOD PRESSURE MEASUREMENT DEVICE**

(30) Priority: 27.03.2006 JP 2006085599
(71) Applicant: Omron Healthcare Co., Ltd., Kyoto 615-0084 (JP)
(72) Inventor: SHIRASAKI, Osamu, Kyoto-shi, Kyoto 615-0084 (JP)
(74) Representative: Wilhelms · Kilian & Partner Patentanwälte
(86) International application number: PCT/JP2007/054902
(87) International publication number: WO 2007/111119

(57) **Abstract**

A connector body (10) includes two latch plates (2,3) which detachably fix a connector body (10) and a connector body (20). The two latch plates (2,3) are configured to be able to fix the connector body (20) to the connector body (10) at a step portion (20c) of the connector body (20). The step portion (20c) is a position which is different from an airtight portion of an O-ring (4), and the O-ring (4) is used to establish airtightness between a fluid path (1c) of a connector chassis (1) and a fluid path (20d) of a connector body (20). Therefore, a blood-pressure measuring device (100) in which a blood-pressure measuring device main body (60) and a cuff (40) are easily connected while damage of a tube or a piping component can be prevented is obtained.

## Description

### TECHNICAL FIELD

The present invention relates to a blood-pressure measuring device, particularly to a blood-pressure measuring device having a connecting structure between a cuff and a blood-pressure measuring device main body.

### BACKGROUND ART

Recently, a blood-pressure meter is widely spread for the purpose of early detection and blood pressure management of lifestyle-related diseases caused by high blood pressure. Usually, in measuring a blood pressure value, a cuff including a fluid bag which compresses an artery is wound around a surface of a living body, and an arterial pressure pulse wave generated in the artery is detected by expanding and contracting the wound fluid bag, thereby measuring the blood pressure value.

As used herein, the cuff shall mean a belt-like structure having a hollow portion which can be wound around a measured region (such as an upper arm and a wrist) of the living body and utilized in measuring the arterial pressure of the arm or leg by injecting a fluid such as gas and a liquid into the hollow portion. Accordingly, the term of the cuff indicates a concept including the fluid bag and winding means for winding the fluid bag around the living body, and sometimes the cuff is called an arm belt or a manchette.

A clinical condition that the blood pressure is increased during sleep is called nocturnal hypertension, and the nocturnal hypertension is regarded as a strong risk factor for the disease of the brain or heart and sudden death. Therefore, the measurement of the blood pressure during sleep is useful for the diagnosis and treatment of the high blood pressure. Usually an Ambulatory Blood Pressure Monitor (ABPM) is used for the blood pressure measurement during sleep. That is, the miniaturized automatic blood-pressure measuring device is always carried, and the cuff connected to the automatic blood-pressure measuring device with a tube is always attached to the upper arm to periodically and automatically measure and record the blood pressure.

However, it is difficult to attach the blood-pressure measuring device main body to nightwear during sleep, and the blood-pressure measuring device main body disturbs the sleep. Therefore, only the blood-pressure measuring device main body is detached from the body, and a subject often sleeps with the blood-pressure measuring device main body placed near the subject. However, when the subject goes to a bathroom in the nighttime, it is necessary to detach the cuff each time. This is troublesome for the subject, and a risk of improperly attaching the cuff is generated when the subject is not familiar with the device. Although the blood-pressure measuring device main body can be carried by hand without detaching the cuff, the subject is inconveniently handful with the blood-pressure measuring device main body, the subject is possibly dangerous to walk depending on an environment.

Recently, some of the home blood-pressure measuring devices have a measuring function during sleep. However, in such cases, because the cuff and the blood-pressure measuring device main body are connected with the tube, it is necessary to attach and detach the cuff each time, or it is necessary to carry the blood-pressure measuring device main body by hand.

When the blood-pressure measuring device main body and the cuff are detached, it is not necessary to carry the blood-pressure measuring device main body. However, in the structure of the conventional blood-pressure measuring device, because the tube is strongly pushed into a piping component in order to prevent air leakage, the tube is not easily extracted from the piping component (see Patent Documents 1 to 3).
[Patent Document 1] Japanese Patent Laid-Open Publication No. 6-154174
[Patent Document 2] Japanese Patent Laid-Open Publication No. 2002-360527
[Patent Document 3] Japanese Patent Laid-Open Publication No. 2000-83912

### DISCLOSURE OF THE INVENTION

### PROBLEMS TO BE SOLVED BY THE INVENTION

It is not assumed that the tube is frequently attached and detached in the tube connection of the conventional blood-pressure measuring device. Therefore, when the forced attachment and detachment of the tube to and from the piping component are repeated, the tube is possibly damaged to generate the air leakage or the piping component is possibly damaged. Because usually the surroundings are dark during sleep, it is difficult to attach and detach the tube.

In view of the foregoing, an object of the present invention is to provide a blood-pressure measuring device in which a blood-pressure measuring device main body and a cuff are easily connected while damage of the tube or the piping component can be prevented.

### MEANS FOR SOLVING THE PROBLEMS

In accordance with an aspect of the invention, a blood-pressure measuring device includes a cuff having a fluid bag which can be expanded and contracted; a blood-pressure measuring device main body having an expansion and contraction member for expanding and contracting the fluid bag; a flexible connecting pipe which is connected to the expansion and contraction member; and a connecting member which fluid-couples the connecting pipe and the fluid bag, wherein the connecting member includes a first connector member which has a first fluid path and is connected onto a side of the connecting pipe; a second connector member which has a second fluid path and is connected to the cuff; an airtight member which establishes airtightness between the first fluid path and the second fluid path; and a fixing member which detachably fixes the first connector member and the second connector member. The fixing member fixes at least one of the first and second connector members at a position which is different from the airtight portion of the airtight member.

According to the blood-pressure measuring device of the present invention, the fixing member detachably fixes the connector members to each other at the position which is different from the airtight portion. Therefore, the first and second connector members are attached and detached without applying a load on the airtight member, so that the damage of the airtight member can be prevented in attaching and detaching the first and second connector members. Additionally, the attachment and detachment of the flexible connecting pipe are not required in attaching and detaching the first and second connector members, the damage of the connecting pipe is not generated. Accordingly, the airtightness of the fluid path can be ensured even if the first and second connector members are repeatedly attached and detached. Because the fixing member detachably fixes the connector members to each other at the position which is different from the airtight portion, the main body and the cuff can easily be connected compared with the device in which the airtight position is identical to the fixing position.

In the blood-pressure measuring device according to the present invention, preferably one of the first and second connector members includes a step portion, the fixing member is attached to the other of the first and second connector members, and the fixing member has a mechanical configuration whose size can be changed such that a transition can be made between a state in which the fixing member is engaged with the step portion and a state in which the fixing member is not engaged with the step portion.

Therefore, the fixing member can be engaged with the step portion to fix the first and second connector members to each other.

In the blood-pressure measuring device according to the present invention, preferably the fixing member includes two latch plates which are disposed while being relatively slidable, each of the two latch plates has a hole, and the two latch plates are configured such that a size of an opening where the two holes overlap each other is changed by sliding the two latch plates.

Therefore, the size of the opening can be changed by the simple sliding operation of the two latch plates, and the fixing member can be engaged with the step portion.

In the blood-pressure measuring device according to the present invention, preferably the two latch plates are biased by a biasing member so as to become a first state in which the overlapping of the two holes is decreased, and the two latch plates are relatively slid against a biasing force of the biasing member by an external force so as to become a second state in which the overlapping of the two holes is increased.

Therefore, in the state in which the external force is not applied, the overlapping of the holes is decreased, and the size of the opening where the holes overlap each other is decreased to tighten the connector members, so that the first and second connector members can be fixed to each other. When the external force is applied to increase the overlapping of the two holes, the size of the opening where the two holes overlap each other is increased, which allows the engagement with the connector member to be released. Therefore, the first and second connector members can be attached and detached by the simple sliding operation of the two latch plates.

In the blood-pressure measuring device according to the present invention, preferably the hole made in each of the two latch plates has one of a perfect circular shape and an elliptical shape.

In the blood-pressure measuring device according to the present invention, preferably the fixing member is rotatably attached to one of the first and second connector members, and the fixing member is rotated, and whereby the fixing member fixes the other of the first and second connector members while sandwiching the other of the first and second connector members with one of the first and second connector members.

Therefore, the first and second connector members can easily be attached and detached by rotating the fixing member relative to one of the first and second connector members.

In the blood-pressure measuring device according to the present invention, preferably the fixing member includes a pawl portion which is fixed to one of the first and second connector members, the other of the first and second connector members includes an enlarged diameter portion and a notch portion provided in the enlarged diameter portion, and the pawl portion is engaged with a step portion of the enlarged diameter portion by relatively rotating the first and second connector members after the pawl portion is inserted into the notch portion.

Therefore, the pawl portion is inserted into the notch portion to relatively rotate the first and second connector members, which allows the first and second connector members to be easily fixed. Additionally, the first and second connector members can easily be released by performing the reverse operation.

In the blood-pressure measuring device according to the present invention, preferably at least one of the first connector member, the second connector member, and a portion to which the connecting member of the cuff is fixed has a light emitting function.

Because the connector member or the cuff emits light in the dark, the subject can easily recognize the position of the connector member or the cuff to perform the attaching and detaching operation.

### EFFECTS OF THE INVENTION

Thus, in the blood-pressure measuring device of the present invention, because the fixing member detachably fixes the connector members to each other at the position which is different from the airtight portion, the main body and the cuff can easily be connected to prevent the damage of the tube or the piping component.

### BRIEF DESCRIPTION OF THE DRAWINGS

Fig. 1 is a perspective view schematically showing a configuration of a blood-pressure measuring device according to a first embodiment of the present invention.
Fig. 2 is a view showing a functional block of the blood-pressure measuring device of Fig. 1.
Fig. 3 is a flowchart showing a flow of a blood-pressure measuring process performed by the blood-pressure measuring device according to the first embodiment of the present invention.
Fig. 4 is a partial cutaway plan view schematically showing a configuration of a connecting member of the blood-pressure measuring device according to the first embodiment of the present invention.
Fig. 5 is a side view when viewed from a direction of an arrow A of Fig. 4.
Fig. 6 is a sectional view taken along a line VI-VI of Fig. 4.
Fig. 7 is a sectional view taken along a line VII-VII of Fig. 4.
Fig. 8 is a view showing a configuration of a connector body 20 of the blood-pressure measuring device according to the first embodiment of the present invention.
Fig. 9 is a schematic perspective view showing a state in which the connector body 20 of the blood-pressure measuring device according to the first embodiment of the present invention is attached to a cuff.
Fig. 10 is a partial cutaway plan view schematically showing a configuration of a connecting member of a blood-pressure measuring device according to a second embodiment of the present invention.
Fig. 11 is a side view when viewed from a direction of an arrow A of Fig. 10.
Fig. 12 is a sectional view taken along a line XII-XII of Fig. 10.
Fig. 13 is a sectional view taken along a line XIII-XIII of Fig. 10.
Fig. 14 is a view showing a configuration of a connector body 20 of the blood-pressure measuring device according to the second embodiment of the present invention.
Fig. 15 is a sectional view schematically showing configurations of a connecting member and a part of a cuff in a blood-pressure measuring device according to a third embodiment of the present invention.
Fig. 16 is a schematic sectional view showing a state in which connector bodies are fixed to each other in the blood-pressure measuring device of Fig. 15.
Fig. 17 is a sectional view schematically showing configurations of a connecting member and a part of a cuff in a blood-pressure measuring device according to a fourth embodiment of the present invention.
Fig. 18 is a plan view showing a connector body 220 of Fig. 17.
Fig. 19 is a schematic sectional view showing a configuration (a) in which the connector body 20 is attached to an outer cloth and a configuration (b) in which the connector body 20 is attached to a curler in the blood-pressure measuring device according to the first and second embodiments of the present invention.
Fig. 20 is a schematic sectional view showing a configuration in which a base is attached to a curler in the blood-pressure measuring device according to the third embodiment of the present invention.

### DESCRIPTION OF REFERENCE SYMBOLS

- 1, 201: connector chassis
- 1a, 201a: tube connecting port
- 1 b: hole
- 1c, 20d, 101 c, 120d, 201 c, 220d: fluid path
- 2, 3: latch plate
- 2a, 3a: hole
- 2b, 3b: button portion
- 4, 104, 204: O-ring
- 5: leaf spring
- 10, 20, 110, 120, 210, 220: connector body
- 20a: flange portion
- 20b, 220b: cylindrical portion
- 20c, 220c: step portion
- 30, 130, 230: cuff-side connecting member
- 40: cuff
- 41: air bag
- 42: outer cloth
- 43: inner cloth
- 44: curler
- 50: air tube
- 60: blood-pressure measuring device main body
- 70: main body-side connecting member
- 100: blood-pressure measuring device
- 101: base
- 101a: recess portion
- 102: rotary lever
- 102a: pawl portion
- 111: air pump
- 112: air valve
- 113: pressure sensor
- 114: air pump driving circuit
- 115: air valve driving circuit
- 116: amplifier
- 117: converter
- 120a: abutting portion
- 120c: pressed portion
- 121: blood-pressure measuring air system component
- 122: expansion and contraction member
- 131: CPU
- 132: operation unit
- 133: display unit
- 134: memory unit
- 135: timer
- 202: pawl portion
- 220b: notch portion
- 220a: enlarged diameter portion

### BEST MODE FOR CARRYING OUT THE INVENTION

Hereinafter, embodiments of the present invention will be described with reference to the drawings.

### (First Embodiment)

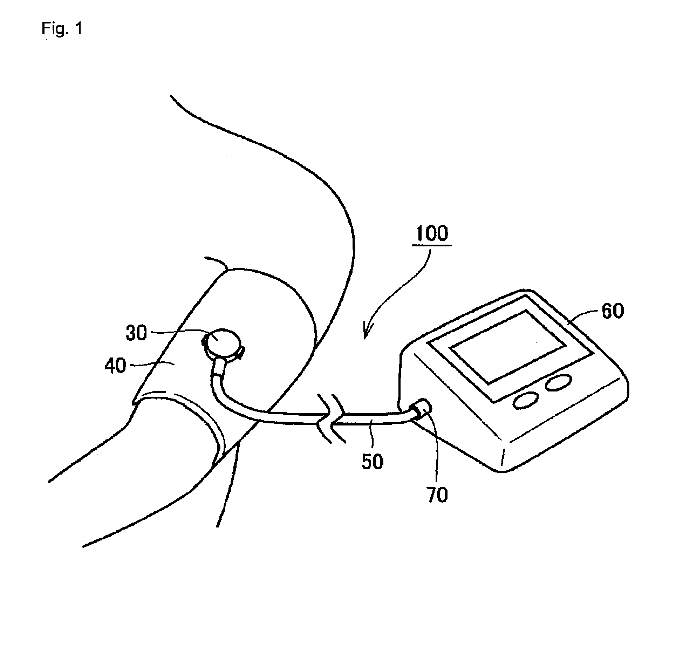

Fig. 1 is a perspective view schematically showing a configuration of a blood-pressure measuring device according to a first embodiment of the present invention. Referring to Fig. 1, a blood-pressure measuring device 100 includes a cuff 40, a blood-pressure measuring device main body 60, an air tube 50, a cuff-side connecting member 30, and a main body-side connecting member 70. The cuff 40 has an air bag therein, and the air bag can be expanded and contracted. For example, the air bag is formed by a rubber bladder. The blood-pressure measuring device main body 60 includes an expansion and contraction member (later-mentioned air pump and air valve) which expands and contracts the air bag. The air tube 50 has flexibility, and the air tube 50 is connected to the expansion and contraction member. The cuff-side connecting member 30 is used to fluid-couple the air tube 50 and the air bag. The main body-side connecting member 70 is used to connect the air tube 50 to the blood-pressure measuring device main body 60. The cuff 40 is attached to an upper arm of a subject. The blood-pressure measuring device main body 60 is detached from a body of the subject rather than being attached to the body during sleep, and the blood-pressure measuring device main body 60 is placed near the subject.

A configuration of a main functional block of the blood-pressure measuring device of the first embodiment will be described below.

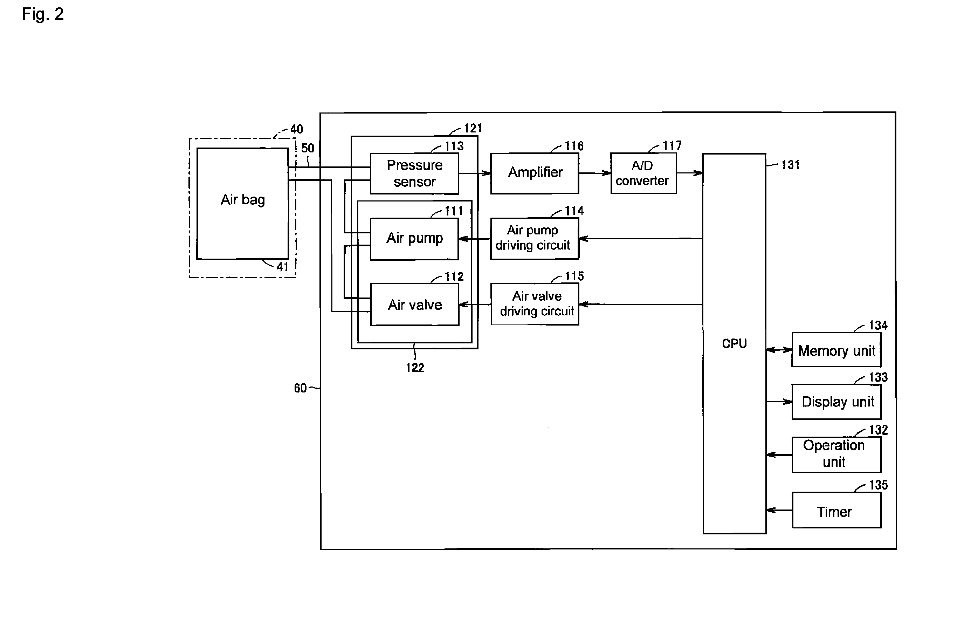

Fig. 2 is a view showing the functional block of the blood-pressure measuring device of Fig. 1. Referring to Fig. 2, a blood-pressure measuring air system component 121 is provided in the blood-pressure measuring device main body 60 in order to supply and evacuate air to and from an air bag 41 accommodated in the cuff 40 through the air tube 50. The blood-pressure measuring air system component 121 includes a pressure sensor 113 which is of pressure detection means for detecting a pressure in the air bag 41 and an air pump 111 and an air valve 112 which are of an expansion and contraction member 122 for expanding and contracting the air bag 41. An amplifier 116, an A/D (Analog/Digital) converter 117, an air pump driving circuit 114, and an air valve driving circuit 115 which are associated with the blood-pressure measuring air system component 121 are provided in the blood-pressure measuring device main body 60.

A CPU (Central Processing Unit) 131, a memory unit 134, a display unit 133, an operation unit 132, and a timer 135 are also provided in the blood-pressure measuring device main body 60. The CPU 131 is used to control and monitor each unit in a concentrated manner. A program for causing the CPU 131 to perform a predetermined operation and various pieces of information such as a measured blood pressure value are stored in the memory unit 134. The display unit 133 displays various pieces of information including a blood pressure measurement result. The operation unit 132 is operated to provide various instructions for the measurement. The timer 135 is used to perform, for example, the blood pressure measurement at a previously set clock time.

The pressure sensor 113 detects a pressure in the air bag 41 (hereinafter referred to as "cuff pressure"), and the pressure sensor 113 supplies a signal corresponding to the detected pressure to an amplifier 116. The air pump 111 supplies air to the air bag 41. The air valve 112 is closed when the pressure in the air bag 41 is maintained, and the air valve 112 is opened when the air in the air bag 41 is discharged. The A/D converter 117 digitizes an analog signal supplied from the amplifier 116, and the A/D converter 117 supplies the digital signal to the CPU 131. The air pump driving circuit 114 controls drive of the air pump 111 based on a control signal supplied from the CPU 131. The air valve driving circuit 115 controls the opening and closing of the air valve 112 based on a control signal supplied from the CPU 131.

A flow of a blood-pressure measuring process in the blood-pressure measuring device of the first embodiment will be described below.

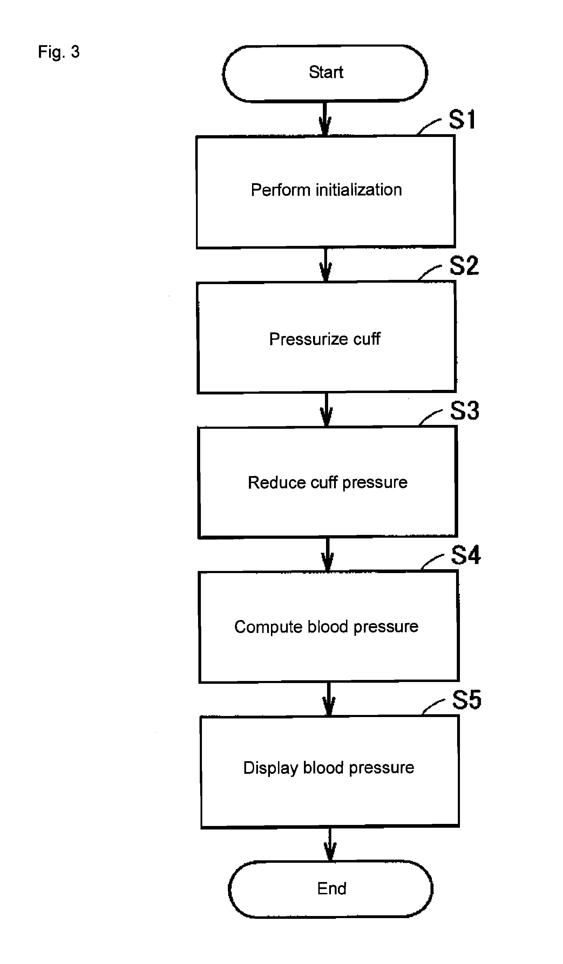

Fig. 3 is a flowchart showing the flow of the blood-pressure measuring process performed by the blood-pressure measuring device according to the first embodiment of the present invention. The program following the flowchart of Fig. 3 is previously stored in the memory unit 134 of Fig. 2, and the CPU 131 reads the program from the memory unit 134 and executes the program to realize the blood pressure measuring process.

Referring to Figs. 2 and 3, when the subject manipulates an operation button of the operation unit 132 of the blood-pressure measuring device 100 to turn on the power, the blood-pressure measuring device is initialized and the blood-pressure measuring device makes a transition to a measurable state (Step S1). When predetermined conditions are prepared to measure the blood pressure, the CPU 131 starts the drive of the air pump 111 to gradually increase the cuff pressure of the air bag 41 (Step S2). In a process of gradually increasing the cuff pressure, when the cuff pressure reaches a predetermined level necessary to measure the blood pressure, the CPU 131 stops the air pump 111, then gradually opens the closed air valve 112 to discharge the air in the air bag 41, and gradually reduces the cuff pressure (Step S3).

Then, the CPU 131 computes the blood pressure value (maximum blood pressure value and minimum blood pressure value) by a well-known procedure (Step S4). Specifically, in a process of gradually reducing the cuff pressure, the CPU 131 computes the blood pressure value based on extracted pulse wave information. When the blood pressure value is computed, the computed blood pressure value is displayed on the display unit 103, and the computed blood pressure value is stored in the memory unit 134 along with measurement date and time (Step S5).

The above-described measurement method is performed based on a so-called reduced-pressure measurement method of detecting the pulse wave during the reduced pressure of the air bag. However, obviously a so-called pressurized measurement method of detecting the pulse wave during the pressurization of the air bag can be adopted. In measuring the blood pressure during sleep, measurement start time is previously fed into the memory unit 134 by operating the operation unit 132, and the blood pressure measurement may be started from the measurement start time by the timer 135.

In the blood pressure measurement during sleep, a determination whether or not body motion exists is made based on a signal obtained from a vibration sensor attached to bedclothing, and the determination that the subject is in the sleep state may be made when the body motion is not recognized for a predetermined time, thereby starting the blood pressure measurement.

A configuration of the connecting member in the blood-pressure measuring device of the first embodiment will be described below.

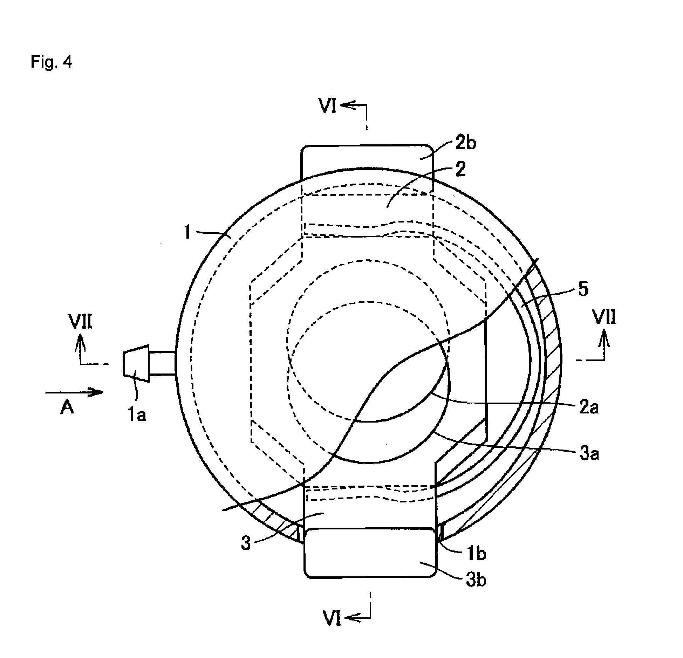

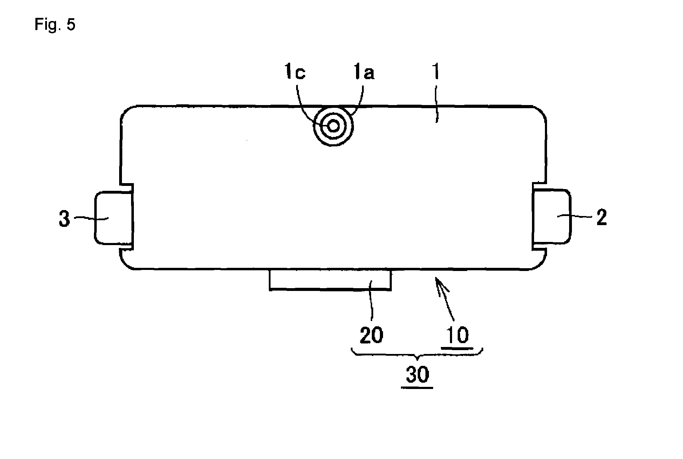

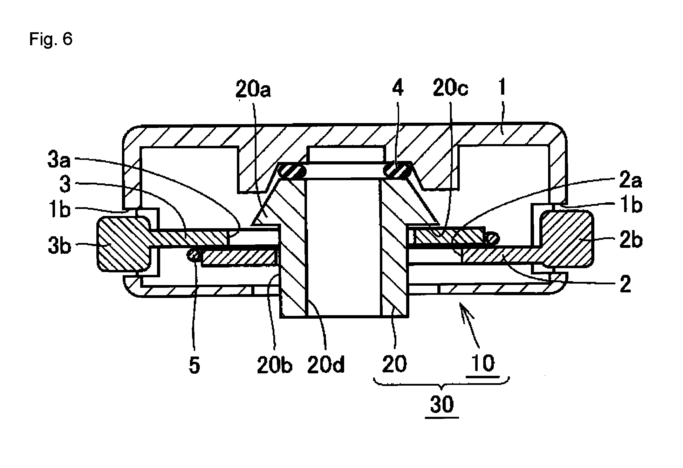

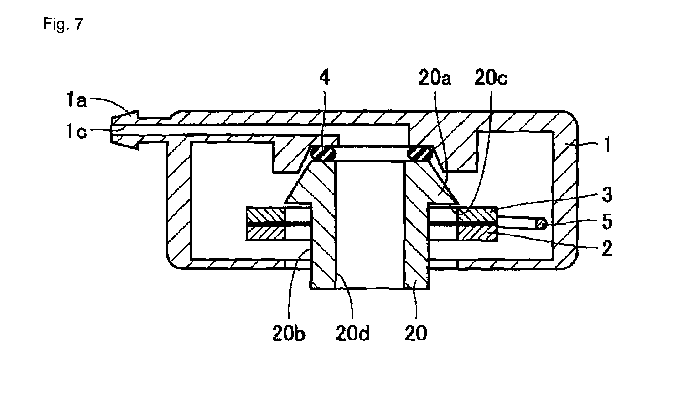

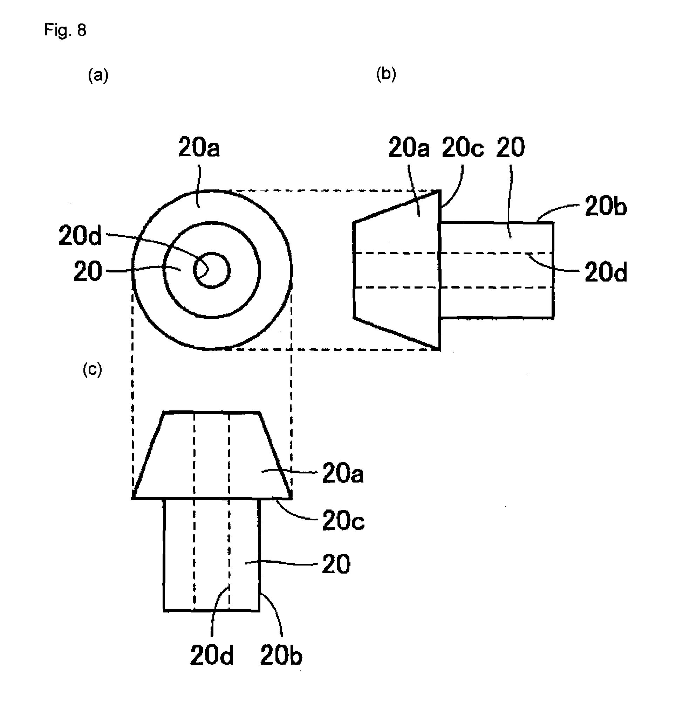

Fig. 4 is a partial cutaway plan view schematically showing the configuration of the connecting member of the blood-pressure measuring device according to the first embodiment of the present invention. Fig. 5 is a side view showing the connecting member when viewed from a direction of an arrow A of Fig. 4, Fig. 6 is a sectional view taken along a line VI-VI of Fig. 4, and Fig. 7 is a sectional view taken along a line VII-VII of Fig. 4. Fig. 8 is a view showing a configuration of a connector body 20 of the blood-pressure measuring device according to the first embodiment of the present invention.

Referring to Figs. 4 to 7, the connecting member 30 includes a connector body 10 connected to the air tube 50 and a connector body (connector member) 20 connected to the air bag of the cuff 40.

Mainly referring to Figs. 6 and 7, the connector body 10 includes a connector chassis (connector member) 1, two latch plates (fixing member) 2 and 3, an O-ring (airtight member) 4, and a leaf spring (biasing member) 5. The connector chassis 1 has a cylindrical shape with a bottom, and the connector chassis 1 includes a tube connecting port 1 a projected from a side face thereof. The tube connecting port 1 a is one to which the air tube 50 of Fig. 1 is connected, and the tube connecting port 1 a includes a fluid path 1 c which is fluid-coupled to the air tube 50. The fluid path 1 c is extended from the tube connecting port 1 a, and is opened to the cylindrical inside.

The O-ring (ring-shaped packing) 4 is disposed so as to encompass surroundings of an inner opening end in the fluid path 1c. The two latch plates 2 and 3 include press button portions 2b and 3b in end portions thereof respectively. Each of the press button portions 2b and 3b is projected outward from a hole 1 b opened to the side face of the connector chassis 1.

Mainly referring to Fig. 4, the two latch plates 2 and 3 include holes 2a and 3a having perfect circular shapes. The two latch plates 2 and 3 are disposed while being relatively slidable in the cylindrical connector chassis 1, and a size of an opening where the two holes 2a and 3a overlap each other can be changed by the slide.

Preferably the press button portions 2b and 3b are disposed with an angle difference of 180° from each other when viewed in a planar manner, and preferably the tube connecting port 1 a is disposed with an angle difference of 90° from each of the press button portions 2b and 3b.

The leaf spring 5 biases the two latch plates 2 and 3 in a slide direction such that the latch plates 2 and 3 act repulsively. Therefore, the two latch plates 2 and 3 are biased so as to become a state (first state) in which the overlapping between the two holes 2a and 3a is decreased. Each of the press button portions 2b and 3b can be pressed inward from the outside of the connector chassis 1 against a biasing force of the leaf spring 5. Therefore, the two latch plates 2 and 3 are relatively slid so as to attract each other, thereby establishing a state (second state) in which the overlapping between the two holes 2a and 3a is increased. The state in which the overlapping is increased shall mean a state in which the holes 2a and 3a having the perfect circular shapes completely overlap each other.

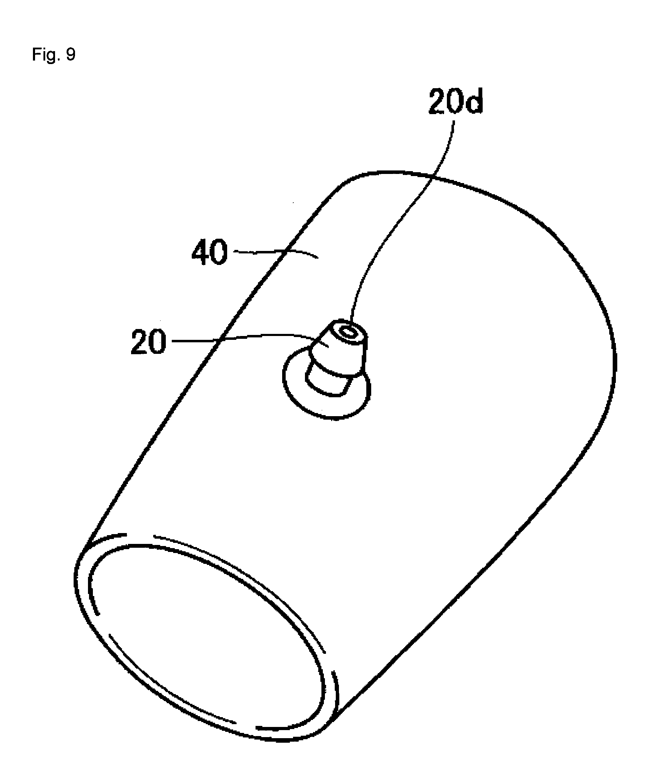

As shown in Fig. 8, the connector body 20 having a plug shape includes a flange portion 20a and a cylindrical portion 20b. The flange portion 20a is formed into a tapered shape in which a size of the flange portion 20a is decreased toward one end of the flange portion 20a, and the cylindrical portion 20b is connected to the other end of the flange portion 20a. A step portion 20c is formed at a boundary of the flange portion 20a and the cylindrical portion 20b. A fluid path 20d which pierces through the flange portion 20a and the cylindrical portion 20b is formed in the connector body 20. As shown in Fig. 9, the connector body 20 is fixed to the cuff 40, and the fluid path 20d of the connector body 20 is communicated with an inner space of the air bag accommodated in the cuff 40.

Mainly referring to Fig. 4, in the state (second state) in which the overlapping of the holes 2a and 3a in the latch plates 2 and 3 is increased, the size of the opening where the holes 2a and 3a overlap each other is set so as to be larger than a maximum size (maximum diameter) of the flange portion 20a. This enables the flange portion 20a to be inserted into the opening where the holes 2a and 3a overlap each other. In the state (first state) in which the overlapping of the holes 2a and 3a in the latch plates 2 and 3 is decreased, the size of the opening where the holes 2a and 3a overlap each other is set so as to be smaller than the maximum size (maximum diameter) of the flange portion 20a. Therefore, when the state (first state) in which the overlapping of the holes 2a and 3a is decreased is established after the flange portion 20a is inserted into the holes 2a and 3a, the latch plate 3 is engaged with the step portion 20c as shown in Fig. 6, which allows the connector body 10 and the connector body 20 to be fixed to each other.

Thus, the fixing member including the two latch plates 2 and 3 are engaged with the step portion 20c of the connector body 20 at the position which is different from the airtight portion of the O-ring 4, thereby fixing the connector body 20 to the connector body 10.

Attaching and detaching operations of the connector bodies 10 and 20 in the connecting member 30 of the first embodiment will be described below.

Before the connector bodies 10 and 20 are attached, the latch plates 2 and 3 are biased in the slide direction by the leaf spring 5 so as to act repulsively. Therefore, the latch plates 2 and 3 are in the state (first state) in which the overlapping of the two holes 2a and 3a is decreased. In the first state, because the size of the opening where the holes 2a and 3a overlap each other is smaller than the maximum size (maximum diameter) of the flange portion 20a, the whole of the flange portion 20a is not inserted into the opening.

In the attaching operation, from the first state, the flange portion 20a of the connector body 20 is pressed against the opening where the holes 2a and 3a overlap each other. Therefore, the opening is gradually pushed and widened by the pressing force of the flange portion 20a. That is, the pressing force of the flange portion 20a relatively slides the two latch plates 2 and 3 so as to attract each other against the biasing force of the leaf spring 5 to make the transition to the state (second state) in which the overlapping of the holes 2a and 3a is increased. The overlapping of the holes 2a and 3a is increased, and the whole of the flange portion 20a is inserted into the opening where the holes 2a and 3a overlap each other at the time the size of the opening reaches the maximum size of the flange portion 20a or more. The latch plates 2 and 3 are slid in the direction in which the latch plates 2 and 3 act repulsively by the biasing force of the leaf spring 5 at the time the cylindrical portion 20b comes to the opening. Therefore, the size of the opening where the holes 2a and 3a overlap each other is smaller than the maximum size of the flange portion 20a, and the latch plates 2 and 3 are engaged with the step portion 20c to fix the connector body 10 and the connector body 20.

In this engagement state, the connector body 10 and the connector body 20 are fixed while a front end of the flange portion 20a presses the connector chassis 1 with the O-ring 4 interposed therebetween. Therefore, because the O-ring 4 is elastically deformed to closely contact the front end of the flange portion 20a, an airtight state is established between the fluid path 1c of the connector chassis 1 and the fluid path 20d of the connector body 20.

In the detaching operation, when the press button portions 2b and 3b projected from the side face of the connector chassis 1 are pushed inward so as to be sandwiched from both sides, the two latch plates 2 and 3 are relatively slid so as to attract each other, thereby establishing the state (second state) in which the overlapping of the holes 2a and 3a is increased. Therefore, the size of the opening where the holes 2a and 3a overlap each other can be set to the maximum size of the flange portion 20a or more, which allows the engagement between the latch plate 3 and the step portion 20c to be released. At the time the engagement is released, the flange portion 20a can be extracted from the opening where the holes 2a and 3a overlap each other, and the connector body 10 can be detached from the connector body 20.

In the first embodiment, the two latch plates 2 and 3 detachably fix the connector body 10 and the connector body 20 at the position (step portion 20c) which is different from the airtight position of the O-ring 4. Therefore, even if damage is generated in the fixing portion due to the repetitive attachment and detachment, the damage is hardly generated in the airtight portion. This enables the damage of the O-ring 4 to be prevented in attaching and detaching the connector body 10 and the connector body 20. Accordingly, the air leakage caused by the damage of the O-ring 4 can be prevented between the fluid path 1 c and the fluid path 20d, and the airtightness can be ensured between the fluid path 1c and the fluid path 20d.

Because the attachment and detachment of the air tube 50 is not required in attaching and detaching the connector body 10 and the connector body 20, the air tube 50 is not damaged by the attachment and detachment of the air tube 50. Accordingly, the air leakage from the air tube 50 is not generated even if the connector body 10 and the connector body 20 are repeatedly attached and detached.

The two latch plates 2 and 3 detachably fix the connector body 10 and the connector body 20 at the position which is different from the airtight position of the O-ring 4. Therefore, it is not necessary to consider the airtightness at the fixing position. Compared with the conventional technique in which the airtight position and the fixing position are located at the same position, the connector body 10 and the connector body 20 can easily be connected.

The size of the opening where the two holes 2a and 3a overlap each other can be controlled by relatively sliding the two latch plates 2 and 3. Therefore, the flange portion 20a can be inserted into and extracted from the opening, and the latch plate 3 is engaged with the step portion 20c after the flange portion 20a is inserted, which allows the connector body 20 to be fixed to the connector body 10. Thus, the connector body 10 and the connector body 20 are attached and detached by the simple operation that the two latch plates 2 and 3 are relatively slid, which allows the subject to attach and detach the connector body 10 and the connector body 20 in one hand.

In fixing the connector body 10 and the connector body 20, it is only necessary to pressurize and press the flange portion 20a against the opening where the two holes 2a and 3a overlap each other, and it is not necessary for the subject to press the press button portions 2b and 3b. In this regard, the connector body 10 and the connector body 20 are easily attached and detached.

The latch plates 2 and 3 are biased by the leaf spring 5 so as to become the state in which the overlapping of the two holes 2a and 3a is decreased. Therefore, the state in which the latch plate 3 is engaged with the step portion 20c is maintained even if the subject does not apply the force to the latch plates 2 and 3 after the flange portion 20a is inserted into the overlapping portion of the two holes 2a and 3a, so that a burden on the subject can be reduced in the fixed state.

### (Second Embodiment)

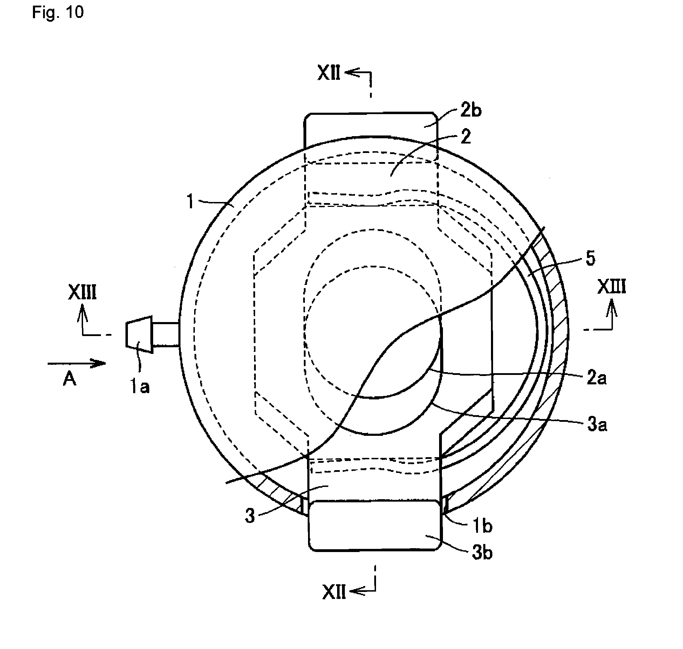

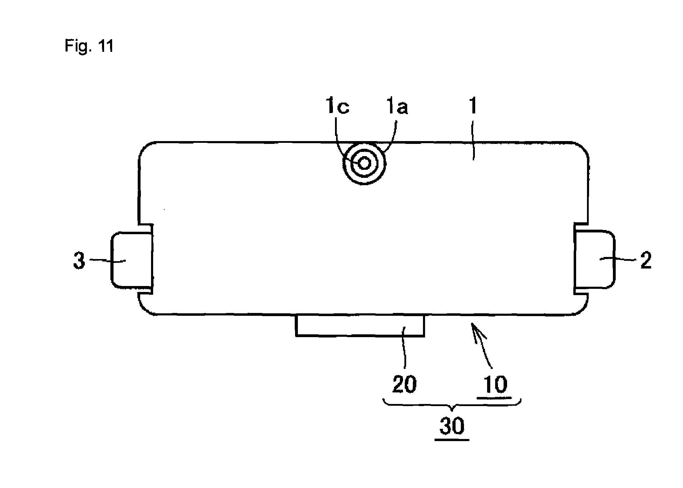

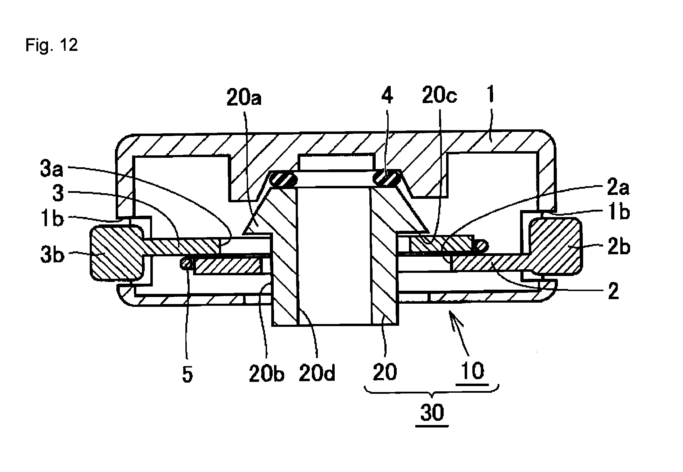

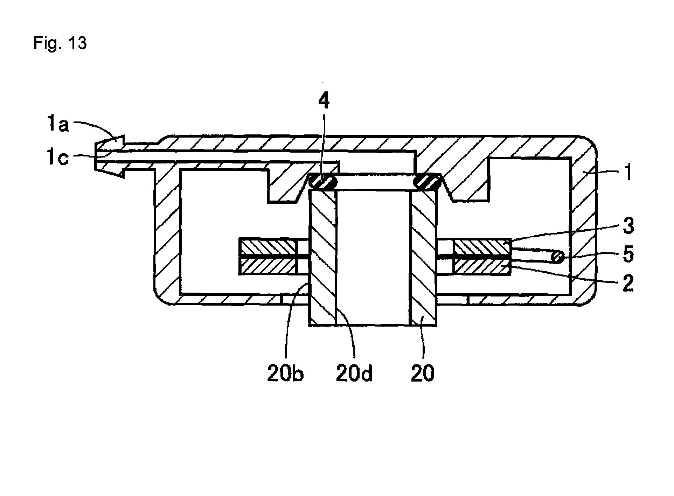

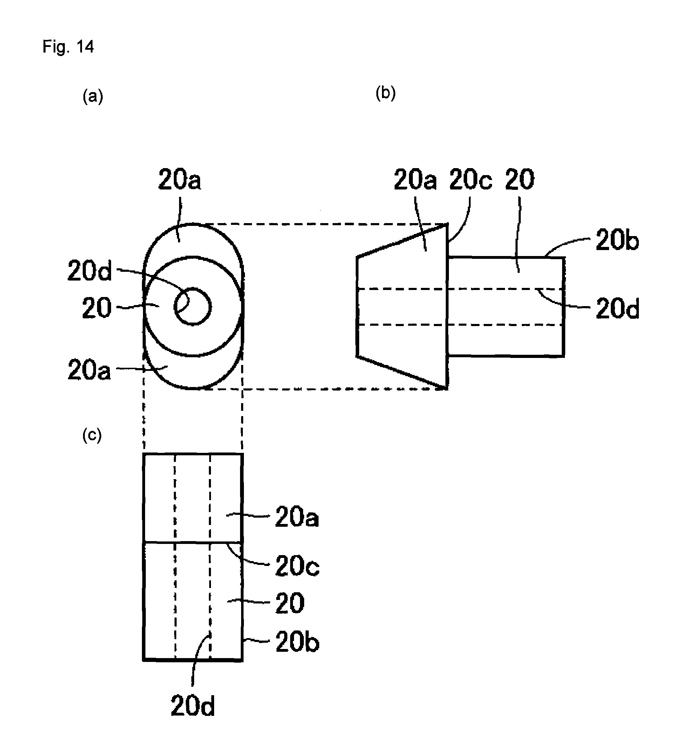

Fig. 10 is a partial cutaway plan view schematically showing a configuration of a connecting member of a blood-pressure measuring device according to a second embodiment of the present invention. Fig. 11 is a side view showing the connecting member when viewed from a direction of an arrow A of Fig. 10, Fig. 12 is a sectional view taken along a line XII-XII of Fig. 10, and Fig. 13 is a sectional view taken along a line XIII-XIII of Fig. 10. Fig. 14 is a view showing a configuration of the connector body 20 of the blood-pressure measuring device according to the second embodiment of the present invention.

Mainly referring to Figs. 10 to 13, the configuration of the second embodiment differs from the configuration of the first embodiment in shapes of the holes 2a and 3a made in the latch plates 2 and 3 and a shape of the flange portion 20a of the connector body 20. For example, the holes 2a and 3a made in the latch plates 2 and 3 have the elliptical shapes. Therefore, the overlapping shape becomes the elliptical shape when the two holes 2a and 3a completely overlap each other.

Mainly referring to Fig. 14, the flange portion 20a of the connector body 20 is provided not in the whole circumference of the cylindrical portion 20b, but on both sides. Therefore, as shown in Fig. 14(a), the flange portion 20a has the elliptical shape when the connector body 20 is viewed from above.

Because other configurations of the second embodiment are substantially similar to those of the first embodiment, the same or corresponding component is designated by the same numeral, and the description is not shown.

The attaching and detaching operations of the connector bodies 10 and 20 in the connecting member 30 of the second embodiment will be described below.

Before the connector bodies 10 and 20 are attached, similarly to the first embodiment, the latch plates 2 and 3 are biased in the slide direction by the leaf spring 5 so as to act repulsively. Therefore, the latch plates 2 and 3 are in the state (first state) in which the overlapping of the two holes 2a and 3a is decreased. In the first state, the whole of the flange portion 20a is not inserted into the opening where the holes 2a and 3a overlap each other.

In the attaching operation, when the press button portions 2b and 3b are pushed inward from the first state so as to be sandwiched from both sides, the two latch plates 2 and 3 are relatively slid so as to attract each other, thereby establishing the state (second state) in which the overlapping of the holes 2a and 3a is increased. Therefore, the opening where the holes 2a and 3a overlap each other becomes the elliptical shape. In the second state, when the flange portion 20a of the connector body 20 is pressed against the opening to rotate the connector body 10, the flange portion 20a is inserted into the opening while the elliptical shape of the opening is matched with the elliptical shape of the flange portion 20a. When the connector body 10 is rotated relative to the connector body 20, the latch plates 2 and 3 are engaged with the step portion 20c to fix the connector body 10 and the connector body 20. When the subject releases a subject's hand from the press button portions 2b and 3b, the latch plates 2 and 3 returns to the state (first state) in which the overlapping of the two holes 2a and 3a is decreased by the biasing force of the leaf spring 5.

In the engagement state, the connector body 10 and the connector body 20 are fixed while the front end of the flange portion 20a presses the connector chassis 1 with the O-ring (airtight member and ring-shaped packing) 4 interposed therebetween. Therefore, because the O-ring 4 is elastically deformed to closely contact the front end of the flange portion 20a, the airtight state is established between the fluid path 1c of the connector chassis 1 and the fluid path 20d of the connector body 20.

In the detaching operation, when the press button portions 2b and 3b projected from the side face of the connector chassis 1 are pushed inward so as to be sandwiched from both sides, the two latch plates 2 and 3 are relatively slid so as to attract each other, thereby establishing the state (second state) in which the overlapping of the holes 2a and 3a is increased. In the second state, when the connector body 10 is rotated relative to the connector body 20, the flange portion 20a can be extracted from the opening in matching the elliptical shape of the flange portion 20a with the elliptical shape of the opening where the two holes 2a and 3a overlap each other, and the connector body 20 can be detached from the connector body 10.

In the second embodiment, similarly to the first embodiment, the two latch plates 2 and 3 detachably fix the connector body 10 and the connector body 20 at the position (step portion 20c) which is different from the airtight position of the O-ring 4. Therefore, the air leakage caused by the damage of the O-ring 4 can be prevented between the fluid path 1 c and the fluid path 20d, and the airtightness can be ensured between the fluid path 1 c and the fluid path 20d.

Because the attachment and detachment of the air tube 50 is not required in attaching and detaching the connector body 10 and the connector body 20, the air leakage from the air tube 50 is hardly generated.

The two latch plates 2 and 3 detachably fix the connector body 10 and the connector body 20 at the position (step portion 20c) which is different from the airtight position of the O-ring 4. Therefore, the connector body 10 and the connector body 20 can easily be connected.

The size of the overlapping portion of the two holes 2a and 3a can be controlled by relatively sliding the two latch plates 2 and 3. Therefore, the connector body 10 and the connector body 20 are attached and detached by the simple sliding operation, which allows the subject to attach and detach the connector body 10 and the connector body 20 in one hand.

The latch plates 2 and 3 are biased by the leaf spring 5 so as to become the state in which the overlapping of the two holes 2a and 3a is decreased, so that the burden on the subject can be reduced in the fixed state.

### (Third Embodiment)

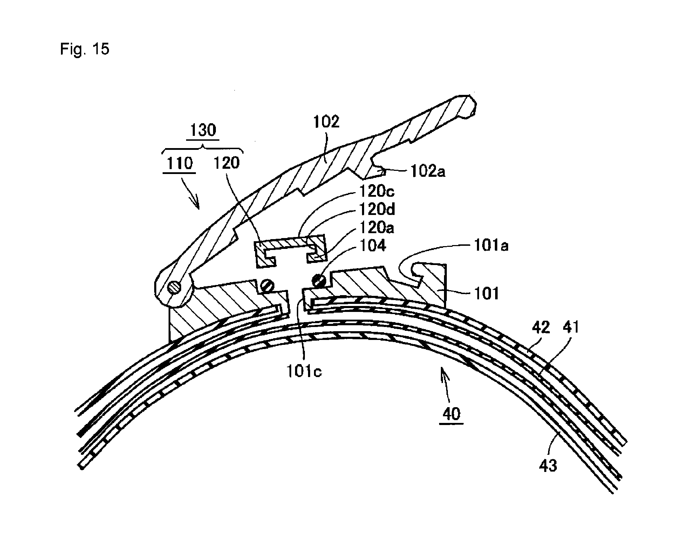

Fig. 15 is a sectional view schematically showing configurations of a connecting member and a part of a cuff in a blood-pressure measuring device according to a third embodiment of the present invention. Referring to Fig. 15, a connecting member 130 includes a connector body 110 and a connector body (connector member) 120.

The connector body 110 includes a base (connector member) 101, a rotary lever (fixing member) 102, and an O-ring (airtight member) 104. The base 101 includes a fluid path 101 c which is communicated with the inner space of the air bag 41 accommodated in the cuff 40. The O-ring (ring-shaped packing) 104 is disposed so as to encompass the surroundings of the open end of the fluid path 101 c. The rotary lever 102 is supported while being rotatable with respect to the base 101. The rotary lever 102 includes a pawl portion 102a, and the pawl portion 102a can be engaged with a recess portion 101 a provided in the base 101.

The connector body 120 includes a fluid path 120d communicated with a fluid path of an air tube (not shown), an abutting portion 120a which can abut on the O-ring 104 at a circumferential edge of the opening of the fluid path 120d, and a pressed portion 120c which is pressed by the rotary lever 102. Thus, the fixing member including the rotary lever 102 presses the pressed portion 120c of the connector body 120 at the position which is different from the airtight portion of the O-ring 104, thereby fixing the connector body 120 to the connector body 110.

The cuff 40 includes the air bag 41, an outer cloth 42, and an inner cloth 43. The air bag 41 is accommodated in the outer cloth 42 and the inner cloth 43.

The attaching and detaching operations of the connector bodies 110 and 120 in the connecting member 130 of the third embodiment will be described below.

Referring to Fig. 15, in the attaching operation, the fluid path 101c of the base 101 and the O-ring 104 are exposed by turning the rotary lever 102 with respect to the base 101. At this point, the connector body 120 is disposed on the base 101 such that the abutting portion 120a of the connector body 120 abuts on the O-ring 104. Then, the rotary lever 102 is turned so as to close the base 101.

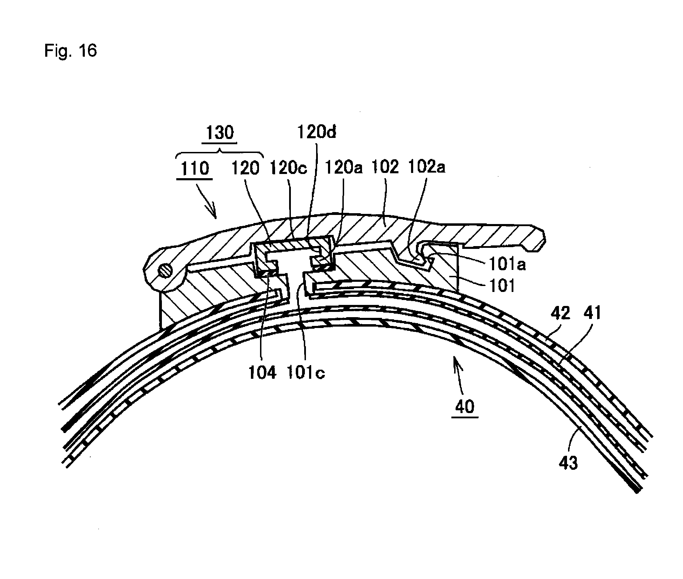

Referring to Fig. 16, the pawl portion 102a of the rotary lever 102 is fitted in the recess portion 101 a of the base 101 by turning the rotary lever 102, thereby fixing the rotary lever 102 to the base 101. This enables the connector body 120 to be attached to the connector body 110. In this state, the rotary lever 102 presses the connector body 120 at the pressed portion 120c, and the abutting portion 120a of the connector body 120 presses the base 101 with the O-ring 104 interposed therebetween. Therefore, because the O-ring 104 is elastically deformed to closely contact the abutting portion 120a, the airtight state is established between the fluid path 101c of the base 101 and the fluid path 120d of the connector body 120.

Referring to Fig. 16, in the detaching operation, the rotary lever 102 is turned to lift up an end portion of the rotary lever 102, whereby the engagement between the pawl portion 102a of the rotary lever 102 and the recess portion 101a of the base 101 is released, and the pressing of the rotary lever 102 against the connector body 120 is also released.

Referring to Fig. 15, the connector body 120 can be detached from the connector body 110 by sufficiently turning the rotary lever 102 in the same direction. Then, the connector body 120 is detached from the base 101.

In the third embodiment, similarly to the first embodiment, the rotary lever 102 detachably fixes the connector body 110 and the connector body 120 at the position (pressed portion 120c) which is different from the airtight position of the O-ring 104. Therefore, the air leakage caused by the damage of the O-ring 104 can be prevented between the fluid path 101c and the fluid path 120d, and the airtightness can be ensured between the fluid path 101c and the fluid path 120d.

Because the attachment and detachment of the air tube is not required in attaching and detaching the connector body 110 and the connector body 120, the air leakage from the air tube is hardly generated.

The rotary lever 102 detachably fixes the connector body 110 and the connector body 120 at the position (pressed portion 120c) which is different from the airtight position of the O-ring 104. Therefore, it is not necessary to consider the airtightness at the fixing position. Compared with the conventional technique in which the airtight position and the fixing position are located at the same position, the connector body 110 and the connector body 120 can easily be connected.

The pressing and non-pressing of the connector body 120 can be controlled by turning the rotary lever 102 with respect to the base 101. Therefore, the connector body 110 and the connector body 120 are attached and detached by the simple sliding operation, which allows the subject to attach and detach the connector body 110 and the connector body 120 in one hand.

### (Fourth Embodiment)

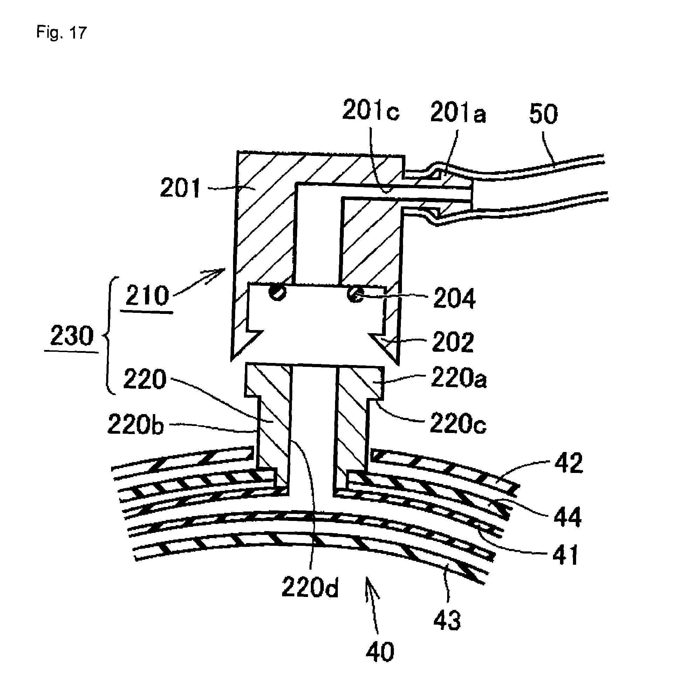

Fig. 17 is a sectional view schematically showing configurations of a connecting member and a part of a cuff in a blood-pressure measuring device according to a fourth embodiment of the present invention. Referring to Fig. 17, a connecting member 230 includes a connector body 210 and a connector body (connector member) 220.

The connector body 210 includes a connector chassis (connector member) 201, two pawl portions (fixing member) 202, and an O-ring (airtight member) 204. The connector chassis 201 has a cylindrical shape with a bottom, and the connector chassis 201 includes a tube connecting port 201 a projected from a side face thereof. The tube connecting port 201 a is one to which the air tube 50 is connected, and the tube connecting port 201 a includes a fluid path 201 c which is fluid-coupled to the air tube 50. The fluid path 201 c is extended from the tube connecting port 1a to the cylindrical inside and the fluid path 201c is opened to the cylindrical lower portion.

The O-ring (ring-shaped packing) 204 is disposed so as to encompass the surroundings of at the open end in a lower portion of the fluid path 201 c. The two pawl portions 202 are provided so as to be extended downward from a lower end of the cylindrical portion of the connector chassis 201.

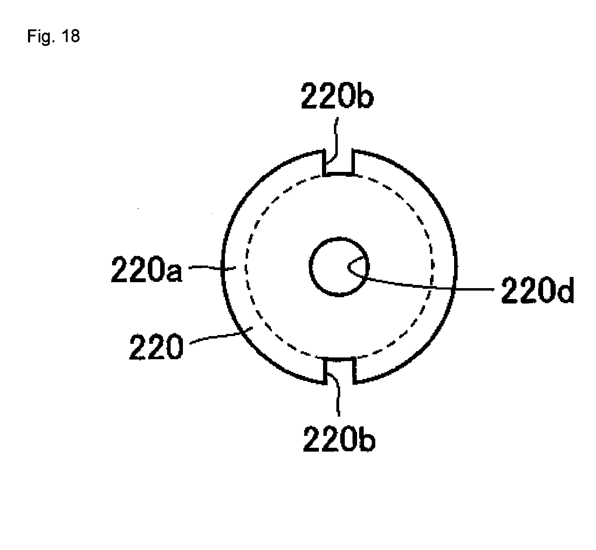

The connector body 220 includes a cylindrical portion 220b and an enlarged diameter portion 220a disposed at a front end of the cylindrical portion 220b. The enlarged diameter portion 220a has a diameter larger than that of the cylindrical portion 220b. A step portion 220c is formed at a boundary of the enlarged diameter portion 220a and the cylindrical portion 220b. A fluid path 220d is formed in the connector body 220, and the fluid path 220d pierces through the enlarged diameter portion 220a and the cylindrical portion 220b. The connector body 220 is, for example, fixed to the cuff 40, and the fluid path 220d of the connector body 220 is communicated with the inner space of the air bag 41 accommodated in the cuff 40. As shown in Fig. 18, the connector body 220 includes the notch portion 220b in the enlarged diameter portion 220a, and the pawl portion 202 can be inserted into the notch portion 220b. The notch portion 220b pierces through the enlarged diameter portion 220a in an axial direction.

Therefore, after the pawl portion 202 is inserted into the notch portion 220b, the connector body 210 is rotated relative to the connector body 220, thereby engaging the pawl portion 202 with the step portion 220c. Thus, the fixing member including the pawl portion 202 is engaged with the step portion 220c of the connector body 220 at the position (step portion 220c) which is different from the airtight portion of the O-ring 204, thereby fixing the connector body 220 to the connector body 210.

The attaching and detaching operations of the connector bodies 210 and 220 in the connecting member 230 of the third embodiment will be described below.

Referring to Figs. 17 and 18, in the attaching operation, after the pawl portion 202 and the notch portion 220b are aligned with each other, the connector body 210 is pressed against the connector body 220. Therefore, the pawl portion 202 is inserted into the notch portion 220b, and a position of the pawl portion 202 reaches a depth of the step portion 220c. At this point, the connector body 210 is rotated relative to the connector body 220 to engage the pawl portion 202 with the step portion 220c. Therefore, the connector body 210 is fixed to the connector body 220.

In the engagement state, the connector body 210 and the connector body 220 are fixed while a front end of the enlarged diameter portion 220a presses the connector chassis 201 with the O-ring 204 interposed therebetween. Because the O-ring 204 is elastically deformed to closely contact the front end of the enlarged diameter portion 220a, the airtight state is established between the fluid path 201 c of the connector chassis 201 and the fluid path 220d of the connector body 220.

In the detaching operation, the pawl portion 202 is moved to the position of the notch portion 220b by rotating the connector body 210 relative to the connector body 220. Therefore, the engagement between the pawl portion 202 and the step portion 220c is released. Then, pawl portion 202 is moved through the notch portion 220b by applying a force to the connector body 220 in a direction in which the connector body 210 is separated from the connector body 220, and finally the pawl portion 202 drops out of the notch portion 220b to detach the connector body 210 from the connector body 220.

In the fourth embodiment, similarly to the first embodiment, the pawl portion 202 detachably fixes the connector body 210 and the connector body 220 at the position (step portion 220c) which is different from the airtight position of the O-ring 204. Therefore, the air leakage caused by the damage of the O-ring 204 can be prevented between the fluid path 201 c and the fluid path 220d, and the airtightness can be ensured between the fluid path 201 c and the fluid path 220d.

Because the attachment and detachment of the air tube 50 is not required in attaching and detaching the connector body 210 and the connector body 220, the air leakage from the air tube 50 is hardly generated.

The pawl portion 202 detachably fixes the connector body 110 and the connector body 120 at the position (step portion 220c) which is different from the airtight position of the O-ring 204. Therefore, it is not necessary to consider the airtightness at the fixing position. Compared with the conventional technique in which the airtight position and the fixing position are located at the same position, the connector body 210 and the connector body 220 can easily be connected.

The attaching and detaching operations of the connector body 210 and connector body 220 can be controlled by simply inserting the pawl portion 202 into the notch portion 220b to rotate the pawl portion 202. Therefore, the subject can attach and detach the connector body 210 and the connector body 220 in one hand.

In the first and second embodiments, the connector body 20 is attached onto the cuff side, and the connector body 10 is attached onto the air tube side. Alternatively, the connector body 10 may be attached onto the cuff side while the connector body 20 is attached onto the air tube side.

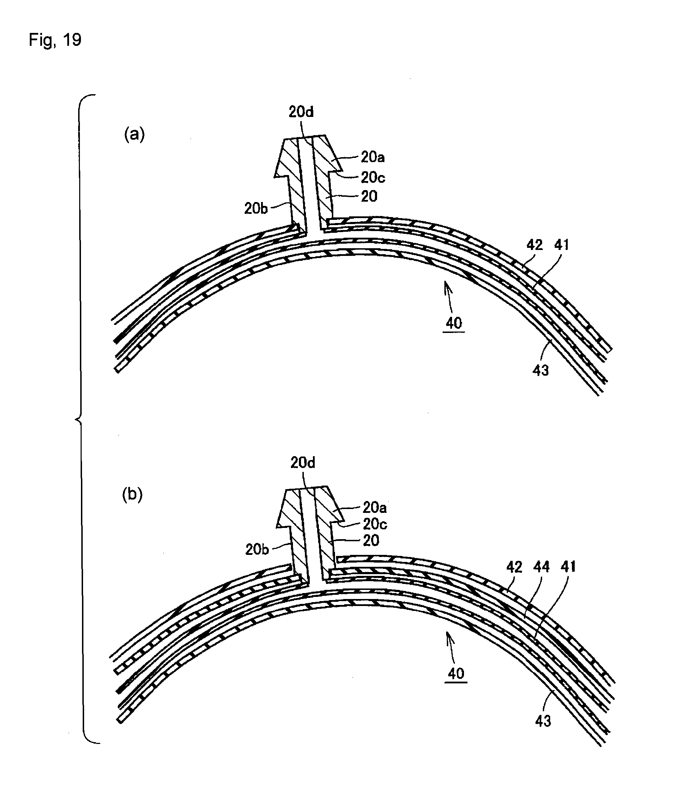

In the first and second embodiments, when the connector body 20 is attached onto the cuff side, the connector body 20 may be fixed to the outer cloth 42 of the cuff 40 as shown in Fig. 19(a), or the connector body 20 may be fixed to a curler 44 added between the outer cloth 42 and the air bag 41 as shown in Fig 19(b). The curler 44 is a flexible member formed by a plate-like member wound in a substantially cylindrical shape, and the curler 44 is made of a resin material such as a polypropylene resin.

In the first and second embodiments, when the connector body 10 is attached onto the cuff side, similarly the connector body 10 may be fixed to the outer cloth of the cuff, or the connector body 10 may be fixed to the curler of the cuff.

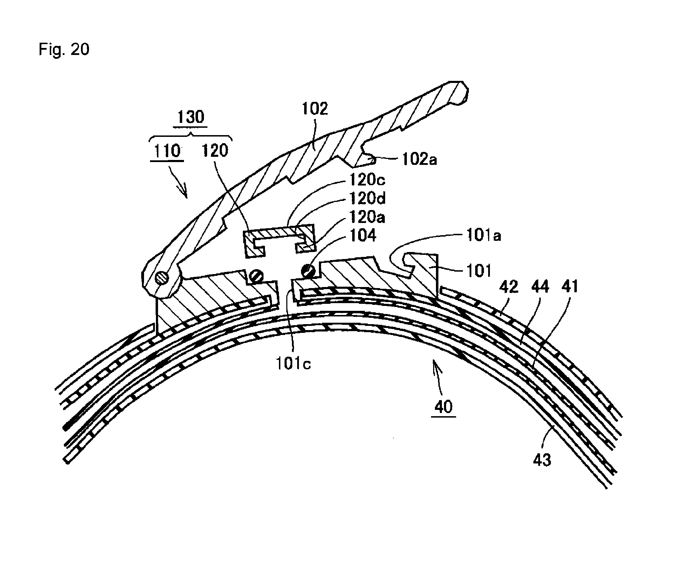

In the third embodiment, the base 101 is fixed to the outer cloth 42 as shown in Fig. 16. Alternatively, the base 101 may be fixed to the curler 44 as shown in Fig. 20.

In the fourth embodiment, the connector body 220 is attached onto the side of the cuff 40 while the connector body 210 is attached onto the side of the air tube 50. Alternatively, the connector body 210 may be attached onto the side of the cuff 40 while the connector body 220 is attached onto the side of the air tube 50.

In the fourth embodiment, when the connector body 220 is attached onto the side of the cuff 40, the connector body 220 may be fixed to the curler 44 of the cuff 40 as shown in Fig. 17, or the connector body 220 may be fixed to the outer cloth 42.

In the fourth embodiment, when the connector body 210 is attached onto the cuff side, similarly the connector body 210 may be fixed to the outer cloth of the cuff, or the connector body 210 may be fixed to the curler of the cuff.

In the first to fourth embodiments, the connector body fixed onto the air tube side and the connector body fixed onto the cuff side can be realized with any plastic material. Preferably the position of the connector bodies can easily be recognized in a dark environment by mixing or coating the luminous paint. Not the connector body fixed onto the cuff side, but a part of the cuff in which the connector body is placed may be coated by the luminous paint or the like, or both the connector body fixed onto the cuff side and a part of the cuff may be coated by the luminous paint or the like. It is only necessary to dispose the element having the light emitting function in the above-described portion, and the element having the light emitting function is not limited to the luminous paint.

In the embodiments, the air bag which is expanded and contracted by injecting the pressurized air to the inside is adopted as an example of the fluid bag. However, the fluid bag is not limited to the air bag, but a gas bag into which gas is injected and a liquid bag into which a liquid is injected may be used as the fluid bag.

The blood-pressure measuring device in which the upper arm is compressed and fixed to measure the blood pressure value is described in the embodiments. The blood-pressure measuring device of the present invention can obviously be applied to a wrist type blood-pressure measuring device, and the blood-pressure measuring device of the present invention can be applied to the blood pressure measurement of any region of the living body such as a front arm, a lower leg, and a body.

The embodiments are described only by way of example, and there is no limitation to the invention. The scope of the invention is shown by not the above-described embodiments but appended claims, and it is to be intended that the meanings equal to the claims and all the modifications within the claims are also included in the invention.

### INDUSTRIAL APPLICABILITY

The present invention relates to the blood-pressure measuring device, and is particularly suitable to the blood-pressure measuring device having the connecting structure between the cuff and the main body.

## Claims

1. A blood-pressure measuring device comprising:
a cuff (40) having a fluid bag (41) which can be expanded and contracted;
a blood-pressure measuring device main body (60) having an expansion and contraction member (111,112) for expanding and contracting said fluid bag;
a flexible connecting pipe (50) which is connected to said expansion and contraction member; and
a connecting member (30) which fluid-couples said connecting pipe and said fluid bag,
wherein said connecting member includes:
a first connector member (10) which has a first fluid path and is connected onto a side of said connecting pipe;
a second connector member (20) which has a second fluid path and is connected to said cuff;
an airtight member (4) which establishes airtightness between said first fluid path and said second fluid path; and
a fixing member (2,3) which detachably fixes said first connector member and said second connector member, and
said fixing member fixes at least one of said first and second connector members at a position which is different from the airtight portion of said airtight member.

2. The blood-pressure measuring device according to claim 1, wherein one of said first and second connector members includes a step portion (20c),
said fixing member is attached to the other of said first and second connector members, and
said fixing member has a mechanical configuration whose size can be changed such that a transition can be made between a state in which said fixing member is engaged with said step portion and a state in which said fixing member is not engaged with said step portion.

3. The blood-pressure measuring device according to claim 2, wherein said fixing member includes two latch plates (2,3) which are disposed while being relatively slidable,
each of said two latch plates has a hole (2a,3a), and
said two latch plates are configured such that a size of an opening where said two holes overlap each other is changed by sliding said two latch plates.

4. The blood-pressure measuring device according to claim 3, wherein said two latch plates is biased by a biasing member (5) so as to become a first state in which the overlapping of said two holes is decreased, and
said two latch plates are relatively slid against a biasing force of said biasing member by an external force so as to become a second state in which the overlapping of said two holes is increased.

5. The blood-pressure measuring device according to claim 3, wherein said hole made in each of said two latch plates has one of a perfect circular shape and an elliptical shape.

6. The blood-pressure measuring device according to claim 1, wherein said fixing member (102) is rotatably attached to one of said first and second connector members (110,120), and
said fixing member (102) is rotated, and whereby said fixing member (102) fixes the other of said first and second connector members while sandwiching the other of said first and second connector members with one of said first and second connector members.

7. The blood-pressure measuring device according to claim 1, wherein said fixing member includes a pawl portion (202) which is fixed to one of said first and second connector members (201,220),
the other of said first and second connector members (201,220) includes an enlarged diameter portion (220a) and a notch portion (220b) provided in said enlarged diameter portion, and
said pawl portion is engaged with a step portion (220c) of said enlarged diameter portion by relatively rotating said first and second connector members after said pawl portion is inserted into said notch portion.

8. The blood-pressure measuring device according to claim 1, wherein at least one of said first connector member, said second connector member, and a portion to which said connecting member of said cuff is fixed has a light emitting function.
